# EUROPEAN PATENT APPLICATION

(11) **EP 4 019 062 A1**
(43) Date of publication of application: **29.06.2022**
(21) Application number: 20874968.9
(22) Date of filing: 05.10.2020
(51) Int. Cl.: A61M 5/14, A61M 1/00

(54) **MEDICAL DEVICE FIXING SYSTEM**

(30) Priority: 07.10.2019 JP 2019184432
(71) Applicant: Senko Medical Instrument Mfg. Co., Ltd., Bunkyo-ku, Tokyo 113-0033 (JP)
(72) Inventor: KATAOKA Daisuke, Tokyo 167-0052 (JP); SHIZUMA Jun, Tokyo 113-0033 (JP)
(74) Representative: Schmidt, Steffen J.
(86) International application number: PCT/JP2020/037778
(87) International publication number: WO 2021/070792

(57) **Abstract**

To provide a connection system for a patient and a human body connection-type medical device that allows a human body of a patient to be connected to a chest drainage unit, a syringe pump, or an infusion pump or a biological observation measurement device (e.g., sphygmomanometer, electrocardiogram) or various other medical devices while allowing the patient to easily move or walk around with the secured safety and function of the medical device. The medical device-fixing system according to the present invention includes a hook section that is engaged with and tightened to a pillar-like support member in a fixed manner and that has an upper face having a concave groove section; and a holder having a lock section that is merely inserted to the concave groove section to provide a locking and fixing function, the holder fixes and retains the medical device. By inserting the lock section of the holder to the groove section of the hook section to achieve a locking and fixing operation therebetween, the medical device is suspended from the pillar-like support member and is connected and fixed thereto.

## Description

### [Technical Field]

The present invention relates to a medical device. In particular, the invention relates to a holder-fixing system that can be used to fix a human body connection-type medical device to a drip infusion pillar, for example.

### [Background Art]

Generally, a medical device (human body connection-type medical device) connected to a human body via a catheter or a probe, for example, includes a chest drainage unit, a syringe pump or an infusion pump, a biological observation measurement device (e.g., sphygmomanometer, electrocardiogram, pulse oximeter) and various other medical devices. The respective medical devices are used in various situations, requiring a special attention to fix the respective medical devices depending on the characteristics of the respective devices. For example, a chest drainage unit, which is a medical device used to drain water or air accumulated in the chest, needs to be fixed based on conditions stricter than those required for a method of fixing a general precision instrument.

For disease states such as a thoracotomy state due to a surgery or external injuries, pneumothorax showing a lung collapse due to holes in a pleura face, or pleural effusion in which a collapsed lung is caused by the pleural effusion or pus accumulated in the chest for example, An approach to expand the collapsed lung by a treatment called a chest drainage has been used where liquid or air accumulated in the chest is discharged from the human body to maintain the chest interior at a negative pressure. This treatment uses a function of a check valve in a water sealing section of a water sealed-type drainage unit. However, any inclination of the water sealing section causes a failure of the backflow prevention function, which causes the air backflow into the chest to undesirably cause the recurrence of the collapsed lung. Thus, attention must be paid not to cause the inclination or falling of the drainage unit. Attention also must be paid to prevent the pleural effusion accumulated in the chest to be sucked into the chest due to the negative pressure in the chest by arranging the chest drainage unit at a position lower than the patient chest.

In a treatment for a bedridden patient, a medical device may be fixed to a bed fence or a floor for example while receiving the treatment. However, in a treatment for a patient moving or walking while receiving the treatment, the medical device must be fixed at an appropriate place during movement or at a moved destination. Furthermore, such a system must avoid a situation where an inappropriate fixing requires the patient to forcedly take an unnecessary rest.

Specifically, consider a case where the patient is instructed to basically receive a drainage treatment on a bed during a drainage period and not to walk due to the reason that the chest drainage unit cannot be fixed appropriately. This instruction seemingly avoids risk and seemingly considers safety. However, such an instruction actually causes a lost treatment opportunity because any rehabilitation cannot be started until the drain is removed and also tramples human dignity under foot because the patient cannot go to the bathroom during the treatment. Thus, it is important to provide options to select an appropriate method of fixing the medical device.

The above chest drainage unit must not be inclined or fallen and must avoid any impact or vibration in order to provide its function, thus frequently requiring the assistance by medical staffs.

However, such an assistance by medical staffs is not always available. Thus, a desired approach is that the patient manages his or her device- fixing system within the scope where the patient can manage the system by himself or herself based on the sufficient understanding of the characteristics of the device. To realize this, an approach is required to provide a simple and easy-to-understand fixing method.

As is the same with to the above chest drainage unit, if a patient is connected to or attached with a syringe pump or an infusion pump, a sphygmomanometer or an electrocardiogram as a biological observation measurement device, or and various other medical devices, the patient also desires, even with the assistance such as a wheelchair, to go to the bathroom if possible than being put in a rest state on a bed. Furthermore, many patients desire a free behaivor of an independent action without any assistance so long as the safety is secured. Thus, various suggestions have been made by the selected documents as shown below in order to provide a fixing method of a human body connection-type medical device that provides economic efficiency and safety.

For example, Patent Literature 1 (JP 2009-233331 A) discloses the following technique to provide a simple and efficient fastener by which a pump or a similar device is removably fixed to a supporter and the pump can be adjusted without requiring the removal of the connection between pump and the supporter.

A clamp is suggested to connect a medical device to a supporter, which includes includes first and second clamp elements substantially opposed to each other and a connection tool to provide a mutual connection between the first and second clamp elements. The first and second clamp elements define a first receptacle to receive one end of the supporter and the second receptacle to receive a fitting structure of the medical device, and at least one of the first and second clamp elements defines a device catch section in the second receptacle. The connection tool allows these clamp elements to be selectively moved between the first position at which one end of the supporter is friction-locked to the fitting structure of the medical device to prevent the relative rotation among the supporter, the clamp, and the medical device and the second position at which these clamp elements are farther from each other than in the case of the first position and the device catch section is positioned so as to prevent the fitting structure of the medical device from being unlocked from the second receptacle so that the medical device can be rotated to the supporter without cancelling the connection to the supporter.

However, the above Patent Literature 1 has an objective of allowing the medical device itself in the fixed state to move freely, thus totally failing to refer to allow a patient attached with a medical device to move and to allow the medical device to move together with the patient. Specifically, Patent Literature 1 does not disclose a technique to allow both of the patient and the medical device to move together safely.

Patent Literature 2 (JP 2016-120396 A) discloses a technique for a connection jig, characterized in comprising: an auxiliary member that can be attached to a bed, a stretcher, or a wheelchair; a first attachment member attached to this auxiliary member in a detachable manner; a second attachment member that can be engaged with a pillar of a drip infusion table; and a connection member to provide the connection between the first attachment member and the second attachment member. The second attachment member has: a frame-shaped main body engaged with the pillar and having an opening through which the pillar can enter or exit; an opening provided in the main body to allow the pillar to enter or exit the main body; and an opening/closing member that is rotatably provided in the main body at the one end side of the opening and that has a tip end locked to an inner face of the main body at the other end side of the opening.

The above technique discloses that a drip infusion pillar having dip infusion goods for a dip infusion treatment (also called a drip infusion pole or a drip infusion table) is connected to a wheelchair to provide a patient with a drip infusion treatment so that the drip infusion pillar can be moved in association with the movement of the wheelchair of the patient. However, this Patent Literature 2 also fails allow the drip infusion pillar to move just in synchronized with the movement of the wheelchair, thus failing to exclude the possibility where the drip infusion pillar may be fallen. This technique also fails to provide the safety to the drip infusion goods as a human body-attached medical device.

Furthermore, the above Patent Literature 1 and Patent Literature 2 disclose a fixing tool suggested to allow a medical device attached and connected to a human body to move by rotation or to change the position in an easy manner. Thus, no description or technique is disclosed with regard to the fall prevention during the movement, the dropout prevention, the safe fixing, the safe release and removal, or the user friendly handling, for example.

### [Citation Prior Art List]

### [Patent Literatures]

[Patent Literature 1] JP 2009-233331 A
[Patent Literature 2] JP 2016-120396 A

### [Summary of the Invention]

### [Problem to be Solved by the Invention]

The present invention has been made in view of solving the above-described technical disadvantages of the prior art. It is an objective of the invention to develop and provide a connection system for a patient and a human body connection-type medical device that allows a medical devices to safely and fully ensure their functions and while the patient being allowed to easily move or walk around, while the human body of the patient is remained attached and connected with such a medical device including a chest drainage unit, a syringe pump, or an infusion pump or a biological observation measurement device (e.g., sphygmomanometer, electrocardiogram) or various other medical devices, for example.

Specifically, the invention has an objective of developing a method and a system to fix a human body connection-type medical device to a device pillar moved together with the patient (e.g., drip infusion pillar or wheelchair).

Consider, the chest drainage unit as a typical example of the human body connection-type medical devices. The system allows a user to hold the chest drainage unit body by one hand, while suspending and fixing the unit on the drip infusion pillar for example. Specifically, the connection system must allow the user to lock the chest drainage unit to the drip infusion pillar by holding the handle of the chest drainage unit with only one hand and without requiring the user to transfer the handle from the one hand to the other hand and to remove the chest drainage unit from the drip infusion pillar by unlocking the chest drainage unit while holding the handle with only one hand and without requiring the user to transfer the handle from the one hand to the other hand. This unlocking method desirably requires two actions (2-step operation) easily carried out only by one hand because a simple one touch action (single operation) may cause a risk of natural unlocking.

Specifically, this is an apparatus system to allow the user to suspend the handle of the frame for fixing the chest drainage unit body on the hook of the pillar for example, to thereby achieve the connection and locking therebetween, while holding the handle. This connection is securely maintained in the fixed state unless the connection is unlocked. In order to unlock the connection, a pushing operation (depression) is performed with a thumb of the hand for example, thereby unlock the chest drainage unit body from the drip infusion pillar, while the handle of the frame for fixing the chest drainage unit body to which the chest drainage body is fixed is being held (two action operation). According to the apparatus system, a series of operations can be performed while gripping the handle of the frame.

It is an objective of the present invention to provide a method of connecting and fixing the chest drainage unit to the drip infusion pillar in a safe and secure manner without requiring a nursing staff or a physician to always accompany the patient while the patient is moving in order to confirm the safety of the function of the chest drainage unit. According to the invention, such a human body connection-type medical device-fixing system is developed and provided by which the medical device and the patient can have remarkably improved safety and simple usability while providing a dramatically improved economic efficiency.

### [Countermeasures for Solving the Problems]

In order to solve the disadvantages as described above, A medical device-fixing system comprising: a hook section that is engaged with and tightened to a pillar-like support member in a fixed manner and that has an upper face with a concave groove section; and a holder having a lock section that is locked and fixed to the hook section by merely inserting the lock section into the concave groove section, thereby fixing and retaining a a medical device; characterized in that: the medical device is suspended from the pillar-like support member, and is connected and fixed thereto by inserting the lock section of the holder to the groove section of the hook section; a locked and fixed state between the concave groove section of the hook section and the lock section is cancelled by a cancellation method positioned in the vicinity of a grip section of the holder, said cancellation is performed by a one touch lock fixing cancellation method based on two actions with one hand, including a push button cancellation method, a slide button cancellation method, an axial rotation cancellation method, an electrical switch cancellation method, an electromagnetic cancellation method, a fingerprint authentication cancellation method or a password cancellation method.

The lock section of the holder of the medical device-fixing system according to the present invention also can be configured so that the lock section of the holder can be locked and fixed to the hook section by merely inserting the locking section into the concave groove section of the hook section, while the medical device being gripped together with the holder with one hand, and the locking is cancelled while being gripped with the one hand without the lock section being transferred from the one hand to the other hand.

According to this invention, while the medical device is continuously attached and connected to the human body of the patient, even when the patient movement is shifted from walking to a wheelchair or from a wheelchair to a stretcher for example, a caregiving staff can instantly move the medical device with one hand accordingly. Thus, the user does not have to fix the tool by turning the line of sight away from the tool or by using both hands for the operation and can easily recognize and handle a change of the symptom of the patient. Thus, the connection system between the patient and the human body connection-type medical device can be provided according to which the function of the safe medical device can be completely secured in order to provide the simple movement or walking of the patient. The hook section is fixed by being engaged and tightened to a pillar such as a drip infusion pillar. This hook section has an upper face including a concave groove section. The concave groove section receives therein the lock section of the holder to which the human body connection-type medical device is fixed for example. Specifically, the drip infusion pillar and the medical device are connected in an integrated manner and are attached to the patient.

Deleted

Specifically, the medical device-fixing system according to the present invention is locked and fixed merely by inserting the lock section of the holder to the concave groove section of the hook section. The lock therebetween can be cancelled by holding the grip section of the holder to which the medical device is fixed to draw the medical device and the holder from the concave groove section by one hand for example by using a push button to cancel the locked state for example. The push button is provided in the vicinity of the grip section of the holder. Thus, the user can grip the holder grip section while depressing the lock button by a finger (e.g., thumb) to cancel the locked state. Specifically, the lock cancellation is achieved by simultaneously performing the two actions composed of an operation to raise the grip section from the concave groove section and an operation to depress the unlock push button by a thumb. This lock cancellation is established only when the operations in different directions are simultaneously performed, thus causing no unintended lock cancellation. This may provide an effective measure to avoid a falling accident.

As described above, the medical device-fixing system according to the present invention has a function to prevent some unexpected accidents by preventing a risk where an unlocking method merely using one touch action (single operation) may cause a natural unlock or falling. Specifically, the two actions (2-step operation) by one hand can remarkably improve the safety.

The lock method and the unlock method may use, in addition to a push button cancellation method, a slide button cancellation method or an axial rotation cancellation method or an electrical switch cancellation method or an electromagnetic cancellation method or a fingerprint authentication cancellation method or a password cancellation method. The lock method and the unlock method can be variously used in the prior art. Thus, an appropriate method may be selected depending on a specific purpose. The medical device-fixing system according to the present invention intends to provide the lock cancellation based on two actions to provide improved safety.

The medical device-fixing system according to the present invention has the lock mechanism in which the locked and fixed state between the concave groove section of the hook section and the lock section is cancelled using the push button. The push button has, at the lower part thereof, a rotation tab section rotated via a spring section (e.g., a compression spring or a tension spring or a plate spring). The rotation tab section is locked and fixed, when the hook section is inserted to the concave groove section, to a receiving section formed in the concave groove section. When the push button is depressed, the rotation tab section is detached from the receiving section to thereby cancel the locked and fixed state based on the push button cancellation method.

Regarding the above lock mechanism, an embodiment will be described later in the "Embodiment for Carrying Out the Invention" section.

The hook section, which is fixed by being engaged and tightened to the pillar-like support member of the medical device-fixing system according to the present invention, has a structure in which a cylindrical hook divided to two sections is engaged with and fixed to the pillar-like support member at the outer periphery. The pillar-like support member also may be a drip infusion pillar or a wheelchair configuration pillar or a bed pillar or a pillar-like support member as a general movable member.

Since the cylindrical hook section is divided to two sections, a cylindrical pillar-like support member may be inserted to the inner diameter to provide strong fixation. The cylindrical support member may be specifically a drip infusion pillar or a wheelchair configuration pillar or a bed pillar that can be moved together with the patient and the medical device. It goes without saying that the cylindrical support member also may be a general movable pillar-like support member.

The medical device of the medical device-fixing system according to the present invention is a medical device attached and connected to a human body, including a chest drainage unit or a syringe pump or an infusion pump or a biological observation measurement device (e.g., sphygmomanometer, electrocardiogram). The holder for fixing the medical device also may be configured to fix the outer periphery section or the rear face section and/or the outer periphery of the lower face section of the medical device in a detachable manner so that the holder is suspended by the support member via the lock section and the hook section and the holder is connected and fixed.

The holder for fixing the medical device to fix the outer periphery section or the rear face section and/or the outer periphery of the lower face section of the medical device in a detachable manner also may include a rotation shoulder having a structure in which one end attached to the holder can be merely axially rotated to fix and retain an end of the outer periphery of the medical device.

The holder for fixing the medical device to fix the outer periphery section or the rear face section and/or the outer periphery of the lower face section of the medical device in a detachable manner also may include a medical device outer periphery storage rail that can fix and retain the medical device.

Specifically, the holder of the medical device-fixing system according to the present invention is a jig to strongly fix a medical device attached and connected to a human body (e.g., a chest drainage unit or a syringe pump or an infusion pump or a biological observation measurement device (e.g., sphygmomanometer, electrocardiogram)) by covering the outer periphery for example to provide a strong integration of the holder and the medical device. The shape of the holder should be determined depending on the outer shape of the medical device to be retained for example. In the case of a medical device having a large bottom face, the holder may have a rail-like shape to retain the bottom face and to provide a slide movement.

In order to allow the holder to retain the medical device, the holder should preferably fix the outer periphery section or the rear face section and/or the outer periphery of the lower face section of the medical device in a detachable manner. Via the lock section and the hook section of the holder, the medical device is suspended on a support member such as a drip infusion pillar or a wheelchair configuration pillar or a bed pillar and is connected and fixed thereto.

The rotation shoulder has a structure in which one end attached to the holder of the medical device-fixing system according to the present invention is merely axially rotated to thereby fix and retain an end of the outer periphery of the medical device. This rotation shoulder will be described later in an embodiment in the "Embodiment for Carrying Out the Invention" section with reference to the drawings.

The present invention is not limited to the above-described embodiments. Various modifications are possible within the scope not deviating from the intention of the present invention. Thus, appropriate embodiments for various applications are all included in the technical scope of the present invention. The equivalent methods using the prior art are also entirely included in the technical concept of the present invention.

### [Effect of the Invention]

According to the present invention, the user can grip the handle of the frame for fixing the chest drainage unit body while suspending the handle on the pillar hook for example to provide the connection and lock therebetween. Furthermore, this connection is maintained in the fixed state unless being unlocked. This apparatus system allows the user to unlock the connection while gripping the handle of the frame to thereby unlock the medical device such as a chest drainage unit body from the drip infusion pillar. The series of operations can be performed while gripping the handle of the frame.

The present invention also provides such an unlocking method by which two actions (2-step operation) by one hand have a function to prevent some accidents in the case of a simple one touch action (single operation) due to an unexpected unlocking (e.g., a risk of a careless unlocking operation or a drainage unit drawn due to a caught tube), thereby remarkably improving the safety.

The present invention provides a safe and secure method to connect and fix a drip infusion pillar without requiring a nursing attendant or a physician to always accompany the patient while the patient is moving in order to secure the safety of the chest drainage unit function. According to the invention, such a medical device-fixing system is developed and provided by which the medical device and the patient can have remarkably-improved safety and simple usability while providing a dramatically-improved economic efficiency.

Specifically, according to the present invention, a system to connect the patient and the human body connection-type medical device can be developed and provided that allows the human body of the patient to be continuously attached and connected to various medical devices (e.g., a chest drainage unit or a syringe pump or an infusion pump or a biological observation measurement device (e.g., sphygmomanometer, electrocardiogram)) while allowing the patient to simply move or walk. This system is safe and can completely secure the function of the medical device.

### [Brief Description of the Drawings]

Fig. 1 is a perspective conceptual diagram illustrating the entirety of a medical device-fixing system according to one embodiment of the present invention.
Fig. 2 is a perspective conceptual diagram illustrating a hook section of the medical device-fixing system according to one embodiment of the present invention.
Fig. 3 is a perspective conceptual diagram illustrating a holder of the medical device-fixing system according to one embodiment of the present invention.
Fig. 4 is a perspective conceptual diagram illustrating the lock section of the holder of the medical device-fixing system according to one embodiment of the present invention.
Fig. 5 is a cross-sectional conceptual diagram illustrating how the lock section and a concave groove section are locked in the medical device-fixing system according to one embodiment of the present invention.
Fig. 6 is a cross-sectional conceptual diagram illustrating how the lock section and the concave groove section are locked in the medical device-fixing system according to one embodiment of the present invention.
Fig. 7 is a cross-sectional conceptual diagram illustrating how the lock section and the concave groove section are locked in the medical device-fixing system according to one embodiment of the present invention.

### [Embodiment for Carrying Out the Invention]

The following section will describe an embodiment to carry out the present invention with reference to the drawings. The following description schematically shows a scope required for the description to achieve the objective of the present invention and will mainly describe the scope of the present invention and will not describe known techniques.

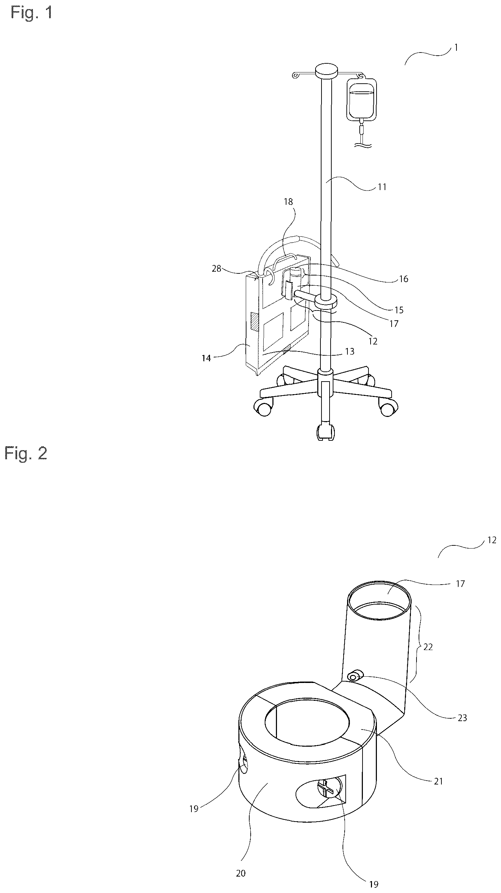

Fig. 1 is a perspective conceptual diagram illustrating the entirety of a medical device-fixing system according to one embodiment of the present invention. As shown in Fig. 1, a medical device-fixing system 1 according to one embodiment of the present invention is configured to include a pillar-like support member 11, a hook section 12, a holder 13, and a medical device 14. The holder 13 is connected to a lock section 15 having a push button 16. The hook section 12 has a concave groove section 17 to which the lock section 15 of the holder 13 is inserted and locked.

As shown in Fig. 1, the hook section 12 is fixed by being engaged and tightened to the outer periphery of the pillar-like support member 11. The pillar-like support member 11 also may be a drip infusion pillar or a wheelchair configuration pillar or a bed pillar or a pillar-like support member as a general movable member. The pillar-like support member 11 in Fig. 1 shows a drip infusion pillar (also may referred to as a portable drip infusion pole or a drip infusion wagon).

The human body connection-type medical device 14 is fixed and held while allowing the outer periphery of the back face to be covered by the holder 13. The medical device 14 is a medical device connected to a human body such as a chest drainage unit or a syringe pump or an infusion pump or a biological observation measurement device (e.g., sphygmomanometer, electrocardiogram). In Fig. 1, the medical device represents the chest drainage unit. The medical device 14 is fixed and held by the holder 13. Thus, when the medical device 1 is not fixed or locked to the hook section 12, the medical device 14 and the holder 13 are potable in an integrated manner. The medical device 14 has a grip section 18 that is gripped for transportation. The grip section 18 also may be attached to the holder 13.

As shown in Fig. 1, the holder 13 has, at an upper part at substantially the center, the lock section 15 that can be cancelled by the push button 16. The lock section 15 is inserted to the concave groove section 17 provided at the upper part at the holder 13 side of the hook section 12 to lock and fix the lock section 15. In Fig. 1, the lock section 15 of the holder 13 is inserted and set in a lock state. Thus, the concave groove section 17 of the hook section 12 is not shown.

The lock section 15 that can be cancelled by the push button 16 also may be based on a slide button cancellation method or an axial rotation cancellation method or an electrical switch cancellation method or an electromagnetic cancellation method or a fingerprint authentication cancellation method or a password cancellation method, as has been described above. These respective lock methods and cancellation methods have been used in the prior art and may be appropriately selected. When the slide button cancellation method is used for example, it is desired that the slide button is provided in the vicinity of a handle section 18 so that the slide button can be slid by a thumb of one hand gripping the handle. Specifically, the cancellation is achieved by the two actions by one hand.

Fig. 2 is a perspective conceptual diagram illustrating the hook section of the medical device-fixing system according to one embodiment of the present invention. As shown in Fig. 2, the medical device-fixing system according to one embodiment of the present invention has the hook section 12 having a hollow cylindrical structure corresponding to the diameter of the pillar-like support member 11 sandwiched by the hook section 12. This hollow cylindrical structure is divided to a hook A section 20 and a hook B section 21 that are engaged with the pillar-like support member 11 for fixation.

In Fig. 2, a screw 19 is used to allow the hook A section 20 and the hook B section 21 to sandwich the pillar-like support member 11 for engagement and fixation. However, neither the pillar-like support member 11 nor the hook section 12 is limited to a circular column-like or cylindrical shape. Any structure may be used so long as the structure allows the hook section 12 to be engaged with and fixed to the pillar-like support member 11 in a secure manner. In addition to the fixation by a screw, various fixing methods according to the conventional method may be used.

As shown in Fig. 2, the hook B section 21 side of the hook section 12 has a lock section receiving section 22 whose upper face has a concave groove section 7 having a cylindrical shape for example. The concave groove section 7 has a structure to which the lock section 15 of the holder 13 described above is inserted. As shown in Fig. 2, the lock section receiving section 22 may have a pin 23 at the outer periphery thereof. The pin 23 may be engaged and positioned in a slit formed in the lock section 15 of the holder 13 to thereby prevent the holder 13 from freely rotating.

In Fig. 2, the concave groove section 7 of the hook section 12 receives the lock section 15 of the holder 13. However, this is one example. Thus, the medical device-fixing system according to the present invention is not limited to a method to lock and engage the hook section and the holder using the insertion to the cylindrical body as shown in Fig. 2. The hook section and the holder may be locked and engaged based on various methods of the prior art. The technical concept of the present invention includes such similar techniques.

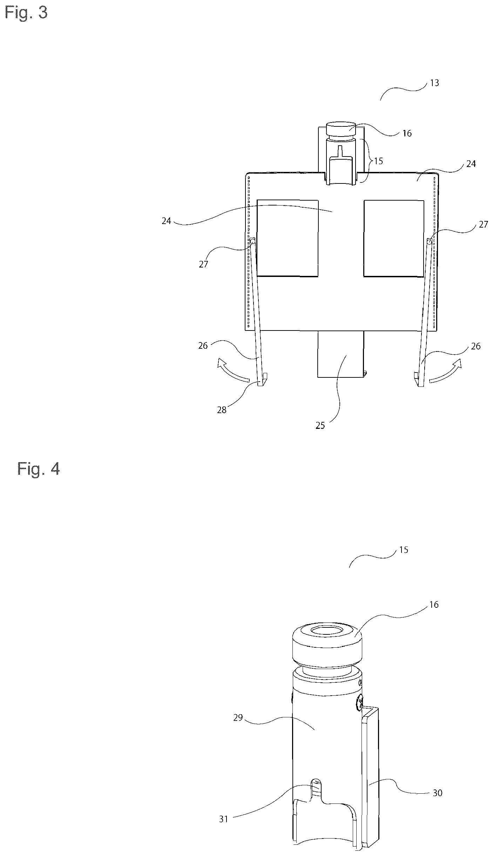

Fig. 3 is a perspective conceptual diagram of the holder of the medical device-fixing system according to one embodiment of the present invention. As shown in Fig. 3, the medical device-fixing system according to one embodiment of the present invention has the holder 13 that is a so-called fixing jig to cover and fix the medical device 14 at the outer periphery circumference. The holder 13 is composed of a holder main body 24, a holder bottom section 25, the lock section 15, and a rotation shoulder section 26. As described above, when the lock state of the lock section 15 is cancelled using a push button, the push button 16 is preferably used as shown in Fig. 3.

As shown in Fig. 3, the holder main body 24 of the holder 13 is configured to function together with the holder bottom section 25 to cover the outer periphery or the bottom face of the medical device depending on the shape of the medical device 14 to securely fix the medical device. The holder main body 24 of the holder 13 is preferably detached in an easy manner. The present invention is not limited to the material of the holder 13. The holder 13 is preferably a sheet metal member for example because the formability and fixation are good.

The medical devices include a medical device attached and connected to a human body such as a chest drainage unit or a syringe pump or an infusion pump or a biological observation measurement device (e.g., sphygmomanometer, electrocardiogram). The holder 13 may have a shape appropriate for to fix the outer periphery of each medical device. Thus, the present invention is not limited to the holder shape as shown in Fig. 3.

As shown in Fig. 3, the holder 13 may have the rotation shoulder section 26 for example. The long bar-like rotation shoulder section 26 is provided for the purpose of fixing the outer periphery of the medical device 14 in a secure manner. For example, the rotation shoulder section 26 is configured so that one end has a rotation center section 27 around which the rotation shoulder section 26 is rotated and the other end has a latch section 28 engaged with the upper end of the outer periphery of the medical device 14 to thereby allow the rotation shoulder section 26 and the holder bottom section 25 to sandwich the medical device 14 in the up-and-down direction, thereby providing a secure fixing.

As described above, the rotation shoulder section 26 and the holder bottom section 25 sandwich the medical device 14 in the up-and-down direction. Since the rotation shoulder section 26 has a main purpose of providing a secure fixing, the direction along which the medical device 14 is fixed by the rotation shoulder section of the medical device-fixing system according to the present invention is not always limited to the up-and-down direction and also may be a lateral left-and-right direction or a front-and-rear direction. Specifically, depending on the outer shape of the medical device to be fixed and secured, the rotation shoulder section may have various different lengths, sizes, or rotation directions for example. Thus, the holder 13 also may have various shapes such as different lengths, sizes, or rotation directions for example depending on the outer shape of the medical device. The technical concept of the present invention includes such similar techniques.

Fig. 4 is a perspective conceptual diagram illustrating the lock section of the holder of the medical device-fixing system according to one embodiment of the present invention. As shown in Fig. 4, the lock section of the holder of the medical device-fixing system according to one embodiment of the present invention is composed of the push button 16, a lock section outer shape section 29, and a holder connection section 30. The internal structure thereof will be described later.

As shown in Fig. 4, the lock section outer shape section 29 of the lock section 15 has a cylindrical structure for example in which the inner periphery receives the lock section receiving section 22 of the hook section 12 inserted from the lower side. The lock section outer shape section 29 has, at the holder 13 side, the holder connection section 30 connected and fixed to the holder 13. The holder connection section 30 is a part that is securely connected and fixed to the upper part of the holder main body 24 at substantially the center. The holder connection section 30 also may not be connected or fixed to the upper part of the holder main body 24 at substantially the center in view of the weight and centroid of the medical device. The slit 31 can be engaged with the pin 23 of the hook section 12 described above and is positioned and fixed to thereby prevent the holder 13 from freely rotating.

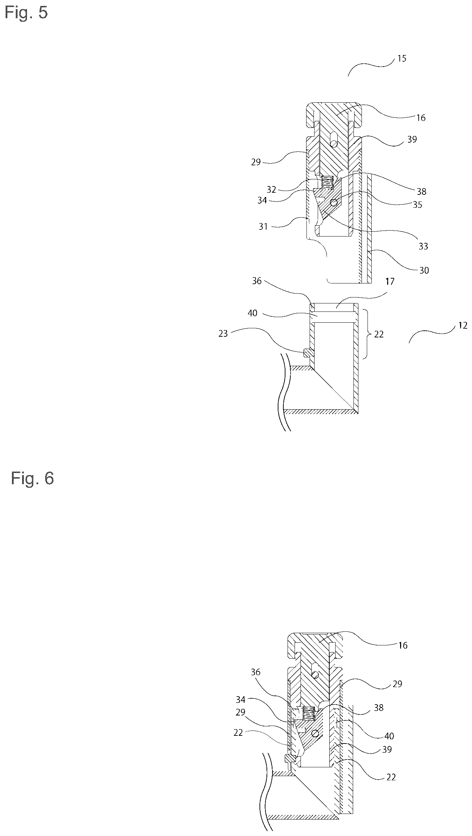

Fig. 5 is a cross-sectional conceptual diagram illustrating how the lock section and the concave groove section of the medical device-fixing system according to one embodiment of the present invention are locked. As shown in Fig. 5, the lock section 15 is mainly composed of an inner cylinder section 39, the lock section outer shape section 29, a spring 32, a rotation tab section 33, and the holder connection section 30. The hook section 12 is composed of the lock section receiving section 22 and the concave groove section 17.

In Fig. 5, the lock section 15 and the hook section 12 both have a cylindrical shape and are mutually engaged and fixed. However, the present invention is not limited to such a cylindrical shape for the engagement. Thus, various other shapes also may be used according to the prior art.

In addition to the above, Fig. 5 illustrates a state immediately before the lock section and the concave groove section are engaged and locked in the medical device-fixing system according to one embodiment of the present invention. The push button 16 of the lock section 15 is movable in the up-and-down direction along the inner diameter of the inner cylinder section 39 via the spring 32. The inner cylinder section 39 is integrated with the lock section outer shape section 29 and the holder connection section 30. The lower part of the push button 16 is linked to the rotation tab section 33 via the spring 34.

As shown in Fig. 5, the rotation tab section 33 is rotated around a rotation center axis 35 and has a tab 34 that is abutted to the lowermost part of the push button 16 and is abutted to the inclined section 38 along the inner diameter of the concave groove section 17 of the hook section 12. The slit 31 of the lock section 15 is configured in a direction along which the slit 31 is engaged with the pin 23 of the hook 12 for a positioning purpose.

Fig. 5 illustrates a state immediately before the lock section and the concave groove section are engaged and locked in the medical device-fixing system according to one embodiment of the present invention. In an order of Fig. 5, Fig. 6, and Fig. 7, the process will be described in which the lock section 15 is inserted to the concave groove section 17 of the hook 12 and is engaged, locked, and fixed after which the engagement and locking are cancelled.

Fig. 6 is a cross-sectional conceptual diagram illustrating how the lock section and the concave groove section are locked in the medical device-fixing system according to one embodiment of the present invention. Fig. 6 illustrates a state in which the lock section 15 is inserted to the concave groove section 17 of the hook section 12 and is engaged, locked, and fixed. When the lock section 15 is inserted to the concave groove section 17 of the hook section 12, the lock section receiving section 22 of the hook section 12 having a cylindrical shape is inserted to a gap between the lock section outer shape section 29 of the lock section 15 having a cylindrical shape larger than the lock section receiving section 22 of the hook section 12 and an inner cylinder body 39 having a cylindrical shape smaller than the lock section receiving section 22 of the hook section 12.

During this insertion, the tab 34 of the rotation tab section 33 of the lock section 15 is downwardly moved along the concave groove section 17 of the inner wall of the lock section receiving section 22 of the hook section 12. On the other hand, an inner wall groove section 40 is formed in the vicinity of the inlet of the inner wall of the lock section receiving section 22 of the hook section 12. Thus, the tab 34 of the rotation tab section 33 of the lock section 15 is inserted to the inner wall groove section 40 and is latched to a tab receiving section 36. In this manner, the lock section is securely engaged with and locked to the concave groove section of the hook section 12 in the medical device-fixing system according to one embodiment of the present invention.

Specifically, according to the medical device-fixing system according to one embodiment of the present invention, the lock section 15 to which the medical device is fixed and engaged and the hook section 12 fixed to the pillar are engaged and fixed by one touch operation to insert the lock section to the hook section, thereby providing a secure locking. In this state, it is completely impossible to upwardly draw the holder 13 and the lock section 15, thus providing a secure and safe locked state.

As described above, according to the present invention, the human body connection-type medical device is connected and fixed to the movable drip infusion pillar for example in a safe and secure manner. The medical device is prevented from being easily detached or fallen from the pillar. The human body connection-type medical device can be safely moved together with the person connected thereto. The pillar-like support member 11 also may be a wheelchair pillar and can be moved safely together with the wheelchair. Alternatively, the pillar-like support member 11 may be a pillar attached to the bed to allow the patient to move together with the bed while the medical device being attached thereto.

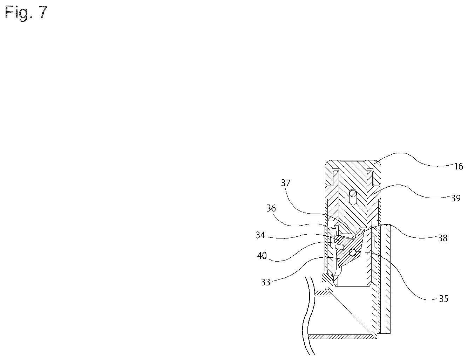

Fig. 7 is a cross-sectional conceptual diagram illustrating how the lock section and the concave groove section are locked in the medical device-fixing system according to one embodiment of the present invention. As shown in Fig. 7, the next step is to cancel the engagement and locking between the lock section 15 and the concave groove section 17 of the hook section 12 by the push button 16.

As shown in Fig. 7, the push button 16 is depressed to cancel the secure engagement and locking between the lock section 15 and the hook section 12. Simultaneously with the downward depression of the spring 32 by the push button 16 along the inner wall of the inner cylinder section 39, a push button convex section 37 of the lowermost part of the push button 16 depresses the inclined section 38 of the rotation tab section 33 in the inclination direction. Then, the rotation tab section 33 is rotated to the right around the rotation center axis 35 (in the case of Fig. 7). Then, the tab 34 of the rotation tab section 33 is detached from the inner wall groove section 40 and the tab receiving section 36 of the concave groove section 17 of the hook section 12 to thereby cancel the engagement, locking and fixing state. Thereafter, the holder 13 and the medical device are upwardly drawn from the hook section 12 of the pillar and are separated from the secure engagement and connection.

As described above, the internal mechanism has been described for the medical device-fixing system according to one embodiment of the present invention to insert, engage, lock and fix the lock section to the concave groove section to subsequently cancel the engagement and locking state. However, this internal mechanism to insert, engage, lock and fix the lock section to the concave groove section to subsequently cancel the engagement and locking state is an example and does not limit the invention. Various engagement and locking mechanisms for the same purpose may be considered in the prior art and are included in the concept of the present invention.

In addition, the technical concept of the present invention in the medical device-fixing system according to one embodiment of the present invention is based not only on the above structure of the engagement and locking mechanism but also on a mechanism in which the medical device is fixed to another drip infusion pillar for example by being easily inserted but is prevented, once locked, from being detached due to the cancellation. Furthermore, the medical device can be detached from the drip infusion pillar for example by cancelling the locked state by one hand. However, a direction along which the medical device holder and the holder are drawn and detached is opposite to a direction along which the push button is depressed by a finger to cancel the locked state. Thus, the invention is based on the technical concept providing the highest safety achieved by the operation composed of the two actions (2-step operation). Thus, the invention suggests a system to completely avoid a careless lock cancellation.

This is a specific example of a safety-first system to connect and fix the human body medical device to the movement pillar as a main objective of the present invention described above. Specifically, the user is allowed to grip the handle of the frame for fixing the chest drainage unit body to suspend the handle on the hook of the pillar as a moving body to thereby connect and lock the handle on the hook. This connection is securely maintained in the fixed state unless any unlock operation is achieved.

In order to unlock the locked state, the user is allowed to grip the handle section of the frame for fixing the chest drainage unit body while depressing the push button by the thumb of the hand for example to perform the two-stage unlocking operation (two actions), thereby unlocking the chest drainage unit body from the drip infusion pillar. The two actions are performed by the hand or the arm moved in different directions. Thus, the locked state cannot be easily cancelled without the intended cancellation operation, thus avoiding a risk and providing a completely-safe apparatus system. This lock cancellation cannot be achieved without the simultaneous execution of the operations in different directions. Thus, no unintended cancellation is caused, thus providing an effective means to prevent a falling accident.

The present invention is not limited to the above-described embodiments. The present invention can be subjected to various modifications within the scope without deviating from the scope of the invention. Such modified embodiments are all included in the technical concept of the present invention. The equivalent methods using the prior art are also included in the technical concept of the present invention.

### [Industrial Applicability]

As described above, according to the invention of this application, a connection system to connect the patient and the human body connection-type medical device can be developed and provided that allows the human body of the patient to be continuously attached and connected to various medical devices (e.g., a chest drainage unit or a syringe pump or an infusion pump or a biological observation measurement device (e.g., sphygmomanometer, electrocardiogram)) while allowing the patient to simply move or walk. This system is safe and can completely secure the function of the medical device.

Specifically, according to the invention of this application, such a medical device-fixing system is realized by which the patient can move with the human body connection-type medical device in an integrated manner with remarkably-improved safety usability while providing a dramatically-improved economic efficiency.

Thus, the present invention is not limited to a medical setting such as a hospital and can be applied to any medical-related facilities. Thus, this application is useful in various industries including the medical field.

### [Description of Reference Numerals]

- 1: Medical device-fixing system
- 11: Pillar-like support member (e.g., drip infusion pole)
- 12: Hook section
- 13: Holder
- 14: Medical device (e.g., chest drainage unit)
- 15: Lock section (holder lock section)
- 16: Push button
- 17: Concave groove section
- 18: Grip section
- 19: Screw
- 20: Hook A section
- 21: Hook B section
- 22: Lock section receiving section
- 23: Pin
- 24: Holder main body
- 25: Holder bottom section
- 26: Rotation shoulder section
- 27: Rotation center section
- 28: Latch section
- 29: Lock section outer shape section
- 30: Holder connection section
- 31: Slit
- 32: Spring
- 33: Rotation tab section
- 34: Tab
- 35: Rotation center axis
- 36: Tab receiving section
- 37: Push button convex section
- 38: Inclined section
- 39: Inner cylinder section
- 40: Inner wall groove section

## Claims

1. A medical device-fixing system comprising:
a hook section that is engaged with and tightened to a pillar-like support member in a fixed manner and that has an upper face with a concave groove section; and a holder having a lock section that is locked and fixed to the hook section by merely inserting the lock section into the concave groove section, thereby fixing and retaining a a medical device; **characterized in that**:
the medical device is suspended from the pillar-like support member, and is connected and fixed thereto by inserting the lock section of the holder to the groove section of the hook section;
a locked and fixed state between the concave groove section of the hook section and the lock section is cancelled by a cancellation method positioned in the vicinity of a grip section of the holder, said cancellation is performed by a one touch lock fixing cancellation method based on two actions with one hand, including a push button cancellation method, a slide button cancellation method, an axial rotation cancellation method, an electrical switch cancellation method, an electromagnetic cancellation method, a fingerprint authentication cancellation method or a password cancellation method.

2. The medical device-fixing system according to claim 1, **characterized in that**: the lock section of the holder can be locked and fixed to the hook section by merely inserting the lock section into the concave groove section of the hook section, while the medical device being gripped together with the holder with one hand, and the locking is cancelled while being gripped with the one hand without the lock section being transferred from the one hand to the other hand.

3. The medical device-fixing system according to claim 1 or 2, **characterized in that**:
the lock fixing cancellation method of the locked and fixed state between the concave groove section of the hook section and the lock section is the lock fixing of the push bottom cancellation method;
the push button has, at a lower part thereof, a rotation tab section rotated via a spring section including a compression spring or a tension spring or a plate spring, the rotation tab section is locked and fixed, when the rotation tag section is inserted to the concave groove section of the hook section, to a receiving section formed in the concave groove section, and when the push button is depressed, the rotation tab section is detached from the receiving section to thereby cancel the locked and fixed state based on the push button cancellation method.

4. The medical device-fixing system according to any one of claims 1 to 3, **characterized in that**: the hook section, which is engaged and fixedly tightened to the pillar-like support member, has a structure in which a cylindrical hook divided to two sections is engaged with and fixed to the pillar-like support member at the outer periphery and the pillar-like support member is a support member for a drip infusion pillar, a wheelchair configuration pillar, a bed pillar, a pillar-like support member of a general movable member.

5. The medical device-fixing system according to any one of claims 1 to 4, **characterized in that**: the medical device is a medical device attached and connected to a human body, including a thorax cavity drainage unit, a syringe pump, an infusion pump or a biological observation measurement device including a sphygmomanometer or an electrocardiogram;
the holder for fixing the medical device is configured to fix the outer periphery section or the rear face section and/or the outer periphery of the lower face section of the medical device in an attachable/detachable manner so that the holder is suspended by and connected and fixed to the support member via the lock section and the hook section.

6. The medical device-fixing system according to any one of claims 1 to 5, **characterized in that**:
the attachable/detachable configuration in which the holder fixes the outer periphery section or the rear face section and/or the outer periphery of the lower face section of the medical device is a configuration with a rotation shoulder having a structure in which one end attached to the holder can be merely axially rotated to fix and retain an end of the outer periphery of the medical device.

7. The medical device-fixing system according to any one of claims 1 to 6, **characterized in that**: the holder for fixing the medical device to fix the outer periphery section or the rear face section and/or the outer periphery of the lower face section of the medical device in a attachable/detachable manner has a medical device outer periphery storage rail that can fix and retain the medical device.
